# EUROPEAN PATENT SPECIFICATION

(11) **EP 3 991 667 B1**
(45) Date of publication and mention of the grant of the patent: **26.08.2026**
(21) Application number: 20831982.2
(22) Date of filing: 29.02.2020
(51) Int. Cl.: A61B 17/122, A61B 17/128

(54) **TISSUE CLAMPING AND CLOSING DEVICE FOR PASSING THROUGH ENDOSCOPE**
GEWEBEKLEMM- UND VERSCHLUSSVORRICHTUNG ZUM DURCHFÜHREN DURCH EIN ENDOSKOP
DISPOSITIF DE SERRAGE ET DE FERMETURE DE TISSU DESTINÉ À PASSER À TRAVERS UN ENDOSCOPE

(30) Priority: 28.06.2019 CN 201910575645
(43) Date of publication of application: 04.05.2022
(73) Proprietor: Micro-Tech (Nanjing) Co., Ltd., Nanjing, Jiangsu 210032 (CN)
(72) Inventor: JIN, Hongyan, Nanjing, Jiangsu 210032 (CN); LENG, Derong, Nanjing, Jiangsu 210032 (CN); LI, Changqing, Nanjing, Jiangsu 210032 (CN); LI, Ning, Nanjing, Jiangsu 210032 (CN); TANG, Zhi, Nanjing, Jiangsu 210032 (CN); XI, Jiefeng, Nanjing, Jiangsu 210032 (CN); WEI, Jianyu, Nanjing, Jiangsu 210032 (CN)
(74) Representative: Zanettin, Gianluigi
(86) International application number: PCT/CN2020/077317
(87) International publication number: WO 2020/258909

(56) References cited:
- EP-A1- 3 603 536
- EP-A1- 3 603 537
- WO-A1-2006/098994
- WO-A1-2018/201406
- WO-A1-2018/228020
- CN-A- 101 116 606
- CN-A- 102 065 780
- CN-A- 102 076 271
- CN-A- 103 330 584
- US-A1- 2004 176 784
- US-A1- 2010 152 753
- US-A1- 2013 006 273
- US-A1- 2018 049 745
- US-A1- 2018 078 262

## Description

### Cross-reference to Related Application

The present disclosure claims the priority to the Chinese patent application with the filing number CN201910575645.0, filed on June 28, 2019 with the Chinese Patent Office, and entitled "Tissue Clamping and Closing Device for Passing Through Endoscope".

### Technical Field

The present disclosure relates to the technical field of medical devices, in particular to a tissue clamping and closing device for passing through an endoscope.

### Background Art

The tissue clamping and closing device is one kind of medical device for treatment of active hemorrhage and mucosal injury of stomach, intestinal tract, etc. When the tissue clamping and closing device is used by a surgeon, a hemostatic clip (also called as clamping and closing assembly) of the tissue clamping and closing device away from an operation end is moved into the stomach, intestinal tract or digestive tract, etc. through an endoscopic clamp channel by using a conveying component, and reach a position of lesion, and the surgeon operates at the operation end of the tissue clamping and closing device to make the hemostatic clip stop bleeding or clamp and close the wound.

The hemostatic clip achieves the purposes of hemostasis and clamping and closing mainly by clamping blood vessels and surrounding tissues, with the hemostasis principle similar to surgical blood vessel suture or ligation. It belongs to a mechanical method, and will not cause coagulation, denaturation, or necrosis of mucosa tissues. After the hemostatic clip of the tissue clamping and closing device clamps and closes the tissues, the local tissues are repaired and form granuloma, and the hemostatic clip will fall off by itself and is discharged out of the body through the digestive tract. Due to the advantages such as small injury, high hemostasis speed, low incidence rate of re-bleeding, few complications, and definite curative effect, such operation mode has become a more common and valuable operation mode in medicine.

Currently, for the tissue closing devices on the market, apart from having the basic tissue clamping and closing function, a part of the tissue closing devices also have a rotating function and a function of opening and closing for many times, and due to the increase of functions of the tissue closing devices, most of the existing tissue closing devices are complex in structure, high in manufacturing and production cost and quality control cost, thus affecting the use stability and reliability of the tissue closing devices. For example, WO2018201406A1 discloses an end portion execution instrument, an end portion execution device, a delivery device and an assembly box; US2013006273A1 discloses a device and a method for through the scope endoscopic hemostatic clipping; US2018049745A1 discloses a hemostasis reloadable clipping device with sleeve engagement; US2018078262A1 discloses a multiple open/close of reloadable clip; US2010152753A1 discloses a hemostatic clipping devices and methods; and WO2018228020A1 discloses a release device for medical tissue clamp.

### Summary

In view of the above-mentioned problems, the present disclosure provides a tissue clamping and closing device for passing through an endoscope. On the basis of ensuring that it has the rotating function and the function of opening and closing for many times, the structure of the tissue clamping and closing device for passing through an endoscope is greatly simplified, its structure becomes quite simple, the production cost and the quality control cost are low, and the use stability and reliability of the tissue clamping and closing device for passing through an endoscope are guaranteed.

In order to achieve the above objectives, and as defined in claim 1, the present invention adopts the following technical solution.

A tissue clamping and closing device for passing through an endoscope, including:
a clip, wherein the clip has at least two clip legs;
a control wire, wherein the control wire is detachably (separably) connected to a through hole at a proximal end of the clip;
an axial sheath, wherein the axial sheath is provided around the control wire;
a sleeve, wherein the sleeve is configured to receive a proximal end portion of the clip and is detachably connected to a distal end of the axial sheath;
a retainer; and
a control mechanism, wherein the control mechanism is connected to the proximal end of the axial sheath and to a proximal end of the control wire;
the control wire is capable of controlling to open or close at least two clip legs of the clip by the control mechanism.

The retainer is a torsion retaining piece provided at a proximal end of the sleeve,
the torsion retaining piece has a connecting portion and a torsion portion, and the connecting portions are provided at two sides of the torsion portion, and connected to the sleeve; the torsion portion is inclined, a proximal end portion of the torsion portion is inclined toward the inside of the sleeve, and a distal end portion of the torsion portion is inclined toward the outside of the sleeve; and
the distal end portion of the torsion portion is configured to maintain the connection between the sleeve and the axial sheath, and is configured to realize separation of the sleeve from the axial sheath by deformation thereof under the action of the control wire.

As a further optional solution of the above tissue clamping and closing device for passing through an endoscope, the axial sheath includes a support seat and a sheath body,
the sleeve is connected to a distal end of the support seat, a proximal end of the support seat is detachably connected to the distal end of the sheath body, and the control mechanism is connected to the proximal end of the sheath body; and
the clamping groove is provided in the distal end of the support seat.

As a further optional solution of the above tissue clamping and closing device for passing through an endoscope, the support seat may be provided therein with a limit portion, and the limit portion in the support seat is configured to abut against the proximal end of the sleeve.

As a further optional solution of the above tissue clamping and closing device for passing through an endoscope, the limit portion in the support seat is a limit step.

As a further optional solution of the above tissue clamping and closing device for passing through an endoscope, an elastic barb is provided at a position of two sides of the proximal end of the clip close to the through hole, a locking slot is provided on the proximal end of the sleeve, and the locking slot is adapted to the elastic barb.

As a further optional solution of the above tissue clamping and closing device for passing through an endoscope, a guide long groove is provided on the sleeve, the guide long groove of the sleeve is provided along a tightening direction of the clip, and the guide long groove of the sleeve is adapted to the elastic barb.

As a further optional solution of the above tissue clamping and closing device for passing through an endoscope, an expansion groove provided along a direction of the elastic barb is provided at two sides of the through hole at the proximal end of the clip.

As a further optional solution of the above tissue clamping and closing device for passing through an endoscope, the retainer is configured to maintain the connection between the sleeve and the axial sheath, and is configured to realize separation of the sleeve from the axial sheath by deformation thereof under the action of the control wire and the clip.

The advantages or principle of the present disclosure is illustrated below.

The tissue clamping and closing device for passing through an endoscope includes: a clip, having at least two clip legs; a control wire, detachably connected to a through hole at a proximal end of the clip; an axial sheath, provided around the control wire; a sleeve, configured to receive a proximal end portion of the clip and detachably connected to a distal end of the axial sheath; a retainer, configured to maintain the connection between the sleeve and the axial sheath, and configured to realize separation of the sleeve from the axial sheath by deformation thereof under the action of the control wire; and a control mechanism, connected to the proximal end of the axial sheath and to a proximal end of the control wire. The control wire can control to open or close at least two clip legs of the clip by the control mechanism. When the tissue clamping and closing device for passing through an endoscope is in use, the surgeon can operate through the control mechanism to pull back the control wire connected with the control mechanism, the control wire drives the clip to move, and enables the clip to be received tightly in the sleeve, thus a clamping and closing operation is completed; if the surgeon considers that a clamped and closed part is not the part he/she wants to clamp, the surgeon can operate again through the control mechanism to push the control wire connected with the control mechanism, then the control wire drives the clip to move, and pushes the clip partially out from the sleeve, so that the clip legs are opened, in this way, multiple times of opening and closing of the tissue clamping and closing device for passing through an endoscope can be realized; if necessary, the surgeon can drive the control wire connected with the control mechanism to rotate by rotating the control mechanism, then the control wire further drives the sleeve and the clip to rotate, thus realizing the rotation of the clamping and closing assembly of the tissue clamping and closing device for passing through an endoscope.

After the tissues are clamped and closed by the clamping and closing assembly of the tissue clamping and closing device for passing through an endoscope, the surgeon can operate through the control mechanism to disengage the control wire from the clip, thus leaving the clamping and closing assembly that clamps and closes the tissues in the human body, and taking the remaining parts of the tissue clamping and closing device for passing through an endoscope from the human body.

On the basis of ensuring that the tissue clamping and closing device for passing through an endoscope has the rotating function and the function of opening and closing for many times, the structure of the tissue clamping and closing device for passing through an endoscope is greatly simplified, and its structure becomes quite simple, thus the production cost and the quality control cost are reduced, so that the use stability and reliability of the tissue clamping and closing device for passing through an endoscope are better guaranteed.

In order to make the above objectives, features, and advantages of the present disclosure more obvious and understandable, preferable embodiments are particularly illustrated in the following to give detailed descriptions in conjunction with the accompanying drawings.

### Brief Description of Drawings

In order to more clearly illustrate technical solutions of embodiments of the present disclosure, accompanying drawings which need to be used in the embodiments will be introduced briefly below, and it should be understood that the accompanying drawings below merely show some embodiments of the present disclosure, therefore, they should not be considered as limitation on the scope, and those ordinarily skilled in the art still could obtain other relevant accompanying drawings according to these accompanying drawings, without using any creative efforts.
FIG. 1 is a structural exploded schematic view of a tissue clamping and closing device for passing through an endoscope in Embodiment 1 of the present disclosure; this embodiment not forming part of the invention;
FIG. 2 is a schematic view of an assembly structure of a clip, a sleeve, a release member, an elastic connecting piece, and a support seat in Embodiment 1 of the present disclosure;
FIG. 3 is a partial structural schematic view of the tissue clamping and closing device for passing through an endoscope, in Embodiment 1 of the present disclosure, for clamping and closing tissues;
FIG. 4 is a schematic view of an assembly structure of the clip and the sleeve in Embodiment 1 of the present disclosure;
FIG. 5 is a first structural schematic view of tightening the clip in the sleeve in Embodiment 1 of the present disclosure;
FIG. 6 is a second structural schematic view of tightening the clip in the sleeve in Embodiment 1 of the present disclosure;
FIG. 7 is a structural schematic view of the clip in Embodiment 1 of the present disclosure;
FIG. 8 is a structural schematic view of the sleeve in Embodiment 1 of the present disclosure;
FIG. 9 is a sectional structural schematic view of the release member in Embodiment 1 of the present disclosure;
FIG. 10 is a sectional structural schematic view of a control wire in Embodiment 1 of the present disclosure;
FIG. 11 is a structural schematic view of the elastic connecting piece in Embodiment 1 of the present disclosure in a natural state;
FIG. 12 is a structural schematic view of the elastic connecting piece in Embodiment 1 of the present disclosure in a stressed state;
FIG. 13 is a structural schematic view of another elastic connecting piece in Embodiment 1 of the present disclosure in a natural state;
FIG. 14 is a structural schematic view of another elastic connecting piece in Embodiment 1 of the present disclosure in a stressed state;
FIG. 15 is a schematic view of another assembly structure of the clip, the sleeve, the release member, the elastic connecting piece, and the support seat in Embodiment 1 of the present disclosure;
FIG. 16 is a sectional structural schematic view of the support seat in Embodiment 1 of the present disclosure;
FIG. 17 is a first partial structural schematic view of the tissue clamping and closing device for passing through an endoscope in Embodiment 2 ; this embodiment forming part of the invention;
FIG. 18 is a second partial structural schematic view of the tissue clamping and closing device for passing through an endoscope in Embodiment 2; and
FIG. 19 is a structural schematic view of the sleeve in Embodiment 2.

### Reference signs:

11-clip; 111-through hole; 112-elastic barb; 113-expansion groove; 114-clamping arm section; 115-blocking protrusion; 12-sleeve; 121-mounting hole; 122-locking slot; 123-guide long groove; 124-blocking groove; 13- control wire; 131-release member; 132-dragline; 1311-first convex portion; 1312-second convex portion; 1313-concave portion; 14-elastic connecting piece; 141-bulge; 15-support seat; 151-clamping groove; 16-sheath body; 17-control mechanism;
22-sleeve; 221-locking groove; 222-guide long groove; 223-blocking groove; 224-torsion retaining piece; 2241-connecting portion; 2242-torsion portion.

### Detailed Description of Embodiments

The technical solutions in the embodiments of the present disclosure will be described clearly and completely below in conjunction with the accompanying drawings in the embodiments of the present disclosure, and apparently, some but not all embodiments of the present disclosure are described. Generally, components in the embodiments of the present disclosure, as described and shown in the accompanying drawings herein, may be arranged and designed in various different configurations. Therefore, the detailed description below of the embodiments of the present disclosure provided in the accompanying drawings is not intended to limit the scope of the present disclosure, but merely illustrates chosen embodiments of the present disclosure.

In the present disclosure, orientation or positional relationships indicated by terms such as "upper", "lower", "left", "right", "front", "rear", "top", "bottom", "inner", "outer", "middle", "vertical", "horizontal", "transverse", and "longitudinal" are based on orientation or positional relationships as shown in the drawings. These terms are mainly used to better describe the present disclosure and its embodiments, and are not used to limit that the indicated device, element, or component must be in a specific orientation, or be constructed and operated in a specific orientation.

In addition, some of the above terms may be used to indicate other meanings in addition to the orientation or positional relationships, for example, the term "upper" may also be used to indicate a certain attachment relationship or connection relationship in some cases. For those ordinarily skilled in the art, specific meanings of these terms in the present disclosure could be understood according to specific circumstances.

Besides, terms "install", "set", "provide", "connect", and "join" should be understood in a broad sense. For example, it may be a fixed connection, a detachable connection, or an integral connection; it may be a mechanical connection, and also may be an electrical connection; it may be a direct connection, indirect connection through an intermediary, or inner communication between two devices, elements or components. For those ordinarily skilled in the art, specific meanings of the above-mentioned terms in the present disclosure could be understood according to specific circumstances.

Besides, terms such as "first" and "second" are mainly used to distinguish different devices, elements or components (specific types and structures may be the same or different), rather than indicating or implying the relative importance or quantity of the indicated device, element or component. "Multiple (a plurality of)" means two or more, unless otherwise illustrated.

### Embodiment 1

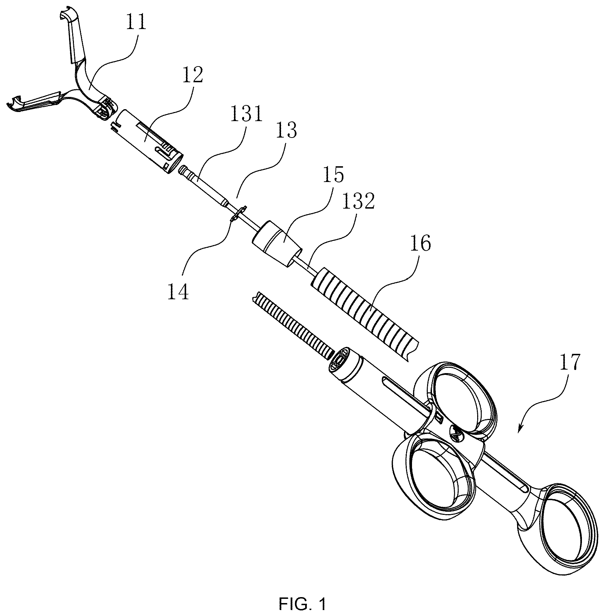

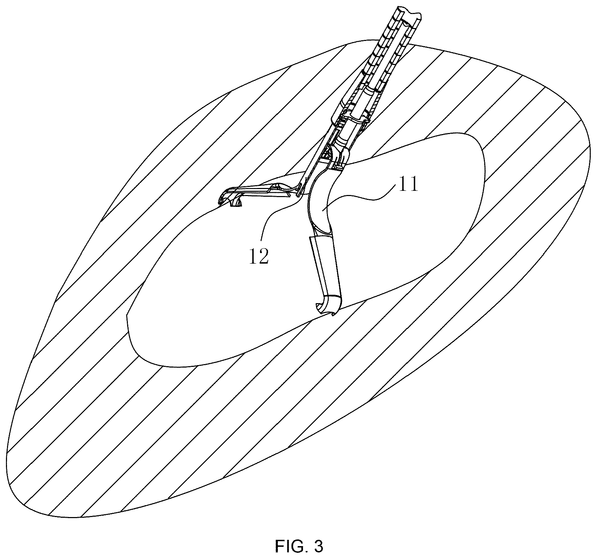

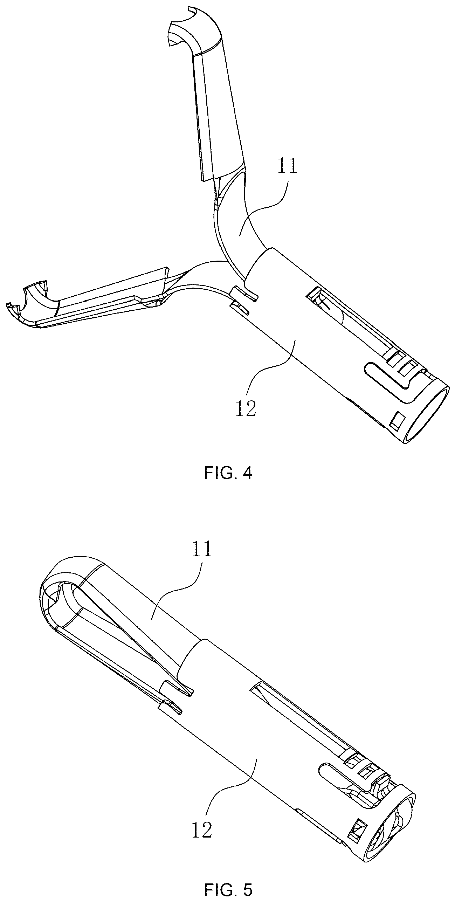

Referring to FIG. 1 to FIG. 16, a tissue clamping and closing device for passing through an endoscope in an embodiment of the present disclosure includes:
a clip 11, wherein the clip 11 has at least two clip legs;
a control wire 13, wherein the control wire 13 is detachably connected to a through hole 111 at a proximal end of the clip 11;
an axial sheath, wherein the axial sheath is provided around the control wire 13;
a sleeve 12, wherein the sleeve 12 is configured to receive a proximal end portion of the clip 11 and is detachably connected to a distal end of the axial sheath;
a retainer, wherein the retainer is configured to maintain the connection between the sleeve 12 and the axial sheath, and configured to realize separation of the sleeve 12 from the axial sheath by deformation thereof under the action of the control wire 13; and
a control mechanism 17, wherein the control mechanism 17 is connected to the proximal end of the axial sheath and to a proximal end of the control wire 13.

The control wire 13 can control to open or close at least two clip legs of the clip 11 by the control mechanism 17.

When the tissue clamping and closing device for passing through an endoscope in the embodiment of the present disclosure is used, a surgeon can operate through the control mechanism 17 to pull back the control wire 13 connected with the control mechanism 17, the control wire 13 drives the clip 11 to move, and tightens the clip 11 in the sleeve 12, so that a clamping and closing operation is completed; if the surgeon considers that a clamped and closed part is not the part he/she wants to clamp, the surgeon can operate again through the control mechanism 17 to push the control wire 13 connected with the control mechanism 17, then the control wire 13 drives the clip 11 to move, and pushes the clip 11 partially out from the sleeve 12, so that the clip legs are opened, in this way, multiple times of opening and closing of the tissue clamping and closing device for passing through an endoscope can be realized; if necessary, the surgeon can drive the control wire 13 connected with the control mechanism 17 to rotate by rotating the control mechanism 17, then the control wire 13 further drives the sleeve 12 and the clip 11 to rotate, thus realizing the rotation of the clamping and closing assembly of the tissue clamping and closing device for passing through an endoscope.

After the tissues are clamped and closed by the clamping and closing assembly of the tissue clamping and closing device for passing through an endoscope, the surgeon can operate through the control mechanism 17 to disengage the control wire 13 from the clip 11, thus leaving the clamping and closing assembly that clamps and closes the tissues in the human body, and taking the remaining parts of the tissue clamping and closing device for passing through an endoscope from the human body.

On the basis of ensuring that the tissue clamping and closing device for passing through an endoscope has the rotating function and the function of opening and closing for many times, the structure of the tissue clamping and closing device for passing through an endoscope is greatly simplified, and its structure becomes quite simple, thus the production cost and the quality control cost are reduced, so that the use stability and reliability of the tissue clamping and closing device for passing through an endoscope are better guaranteed.

Further, the retainer is configured to maintain the connection between the sleeve 12 and the axial sheath, and configured to realize separation of the sleeve 12 from the axial sheath by deformation thereof under the action of the control wire 13 and the clip 11.

When the clamping and closing assembly of the tissue clamping and closing device for passing through an endoscope is used to clamp and close tissues, the surgeon operates through the control mechanism 17 to pull back the control wire 13 connected with the control mechanism 17, then the control wire 13 drives the clip 11 to move, and when the clip 11 is tightened in the sleeve 12, the proximal end portion of the clip 11 will apply a force to the retainer to make the retainer deformed, thereby achieving the separation of the sleeve 12 from the axial sheath.

Referring to FIG. 1 to FIG. 3 and FIG. 16, in the present embodiment, the axial sheath includes a support seat 15 and a sheath body 16.

The sleeve 12 is detachably connected to a distal end of the support seat 15, a proximal end of the support seat 15 is connected to a distal end of the sheath body 16, and the control mechanism 17 is connected to a proximal end of the sheath body 16.

Referring to FIG. 1 to FIG. 3, in the present embodiment, the retainer is an elastic connecting piece 14.

The proximal end of the sleeve 12 is provided with a mounting hole 121, the elastic connecting piece 14 has a bulge 141, the bulge 141 of the elastic connecting piece 14 passes through the mounting hole 121 of the sleeve 12, and the elastic connecting piece 14 is held on the sleeve 12; the distal end of the axial sheath is provided with a clamping groove 151, and the bulge 141 of the elastic connecting piece 14 is embedded into the clamping groove 151 of the axial sheath.

The control wire 13 passes through the elastic connecting piece 14.

When the clamping and closing assembly of the tissue clamping and closing device for passing through an endoscope is used to clamp and close tissues, when the clip 11 is tightened in the sleeve 12, the proximal end portion of the clip 11 will apply an acting force to the elastic connecting piece 14 to make the elastic connecting piece 14 deformed, and as the mounting hole 121 is provided in the proximal end of the sleeve 12, the proximal end portion of the clip 11 acts on the elastic connecting piece 14 with a small acting force, and can only cause simple deformation to the elastic connecting piece 14, but will not cause the elastic connecting piece 14 to escape from the sleeve 12, and the elastic connecting piece 14 will be held on the sleeve 12 all the time.

Further, referring to FIG. 1 to FIG. 3, FIG. 8, and FIG. 16, the mounting hole 121 is provided on each of two sides of the proximal end of the sleeve 12, two sides of the elastic connecting piece 14 each have the bulge 141, and the bulges 141 on the two sides of the elastic connecting piece 14 pass through the mounting holes 121 on the two sides of the proximal end of the sleeve 12.

The clamping groove 151 of the axial sheath is an annular clamping groove, and the bulges 141 on the two sides of the elastic connecting piece 14 are embedded into the annular clamping grooves of the axial sheath.

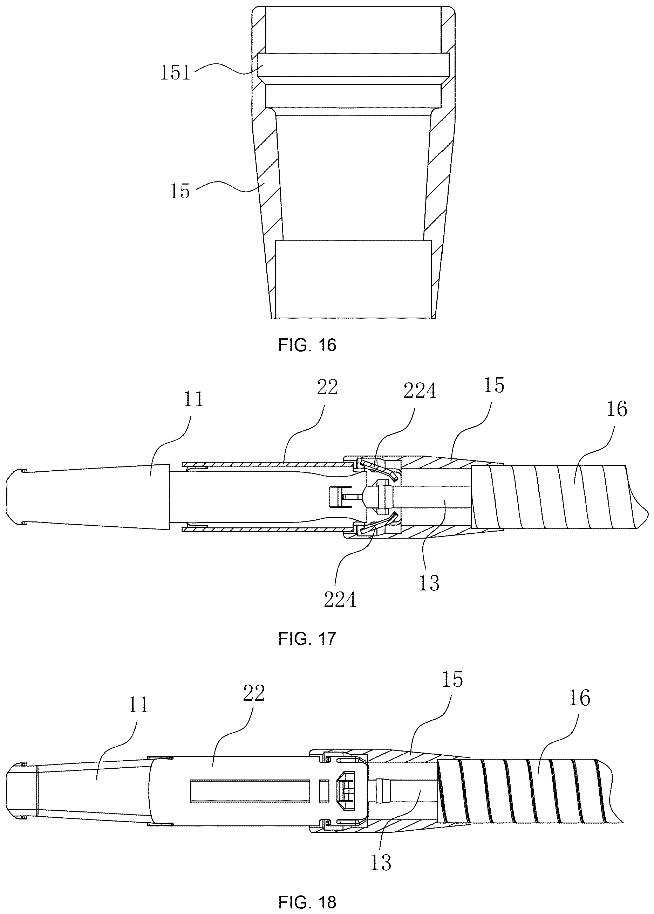

Referring to FIG. 16, in the present embodiment, the clamping groove 151 is provided in the distal end of the support seat 15.

The specific mounting manner of the elastic connecting piece 14 on the sleeve 12 enables the elastic connecting piece 14 to be more stably mounted on the sleeve 12. By adopting an annular clamping groove as the clamping groove 151, the rotation of the elastic connecting piece 14 in the annular clamping groove is not limited, thus facilitating the surgeon to rotate the clamping and closing assembly when using the tissue clamping and closing device for passing through an endoscope, so as to perform surgery or complete surgery more quickly and more effectively, and improve the working efficiency of the surgeon.

Referring to FIG. 1, FIG. 2, and FIG. 9, in the present embodiment, the control wire 13 includes a release member 131 and a dragline 132.

A distal end of the release member 131 is detachably connected to the through hole 111 at the proximal end of the clip 11, a proximal end of the release member 131 is connected to a distal end of the dragline 132, and a proximal end of the dragline 132 is connected to the control mechanism 17.

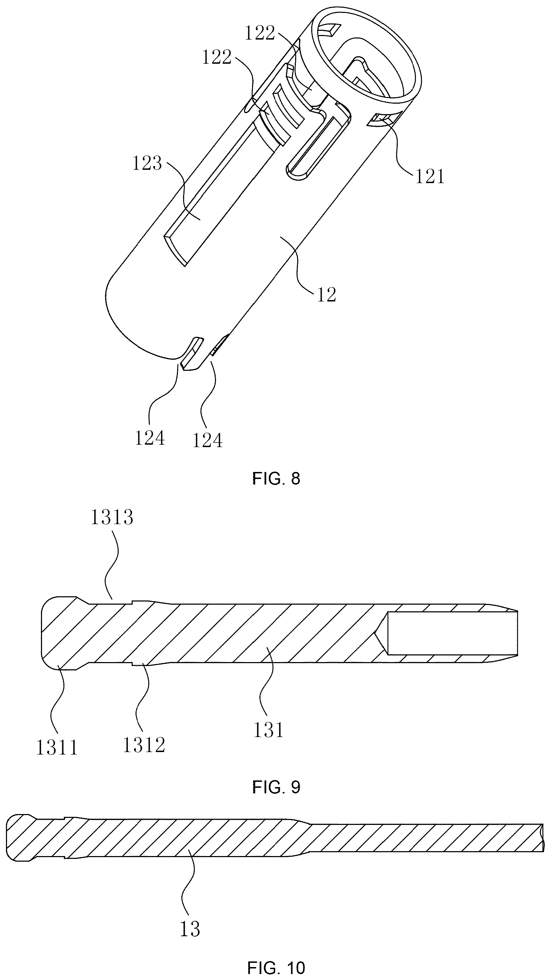

Referring to FIG. 9, in the present embodiment, a connecting hole is provided in a proximal end portion of the release member 131, and the connecting hole in the proximal end portion of the release member 131 is configured to realize the connection between the release member 131 and the dragline 132.

It should be noted that, in another embodiment, referring to FIG. 10, the control wire 13 is an integrally molded structure.

Referring to FIG. 1 to FIG. 16, when being assembled, the tissue clamping and closing device for passing through an endoscope in an embodiment of the present disclosure may be assembled in the following manner.

The bulge 141 of the elastic connecting piece 14 is mounted in the mounting hole 121 of the sleeve 12, the sleeve 12 is mounted on the support seat 15, the clip 11 is mounted on the sleeve 12, one end portion of the sheath body 16 is welded to the support seat 15, the release member 131 is connected to the dragline 132, then the connected release member 131 and dragline 132 pass through the through hole 111 of the clip 11, the distal end portion of the release member 131 is engaged in the through hole 111 of the clip 11, the other end of the dragline 132 is then connected to the control mechanism 17, and finally the other end portion of the sheath body 16 is connected to the control mechanism 17.

It should be noted that, in the present embodiment, the above assembly manner for the tissue clamping and closing device for passing through an endoscope is not unique, and the surgeon or the assembler may assemble the tissue clamping and closing device for passing through an endoscope in the embodiment of the present disclosure in other manners.

Referring to FIG. 1, in the present embodiment, an inner sleeve may be further provided between the sheath body 16 and the dragline 132, and the inner sleeve may fill a gap between the sheath body 16 and the dragline 132, thus reducing the friction between the sheath body 16 and the dragline 132.

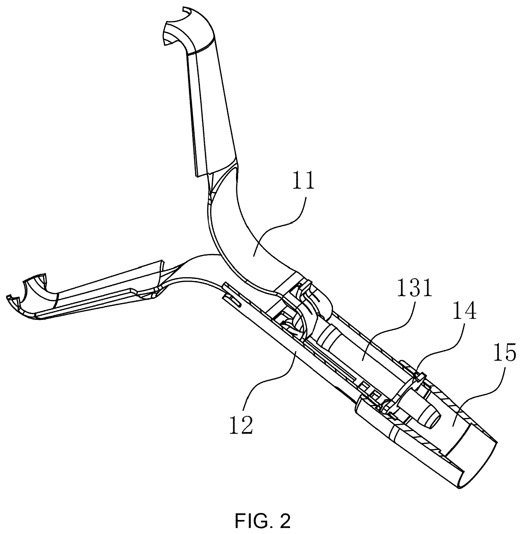

Referring to FIG. 2 and FIG. 9, in the present embodiment, the distal end portion of the release member 131 includes a first convex portion 1311, a concave portion 1313, and a second convex portion 1312.

The first convex portion 1311, the concave portion 1313, and the second convex portion 1312 are provided in order from the distal end portion of the release member 131 to the proximal end portion thereof on the distal end portion of the release member 131.

Specific configuration of the distal end portion structure of the release member 131 enables the distal end portion of the release member 131 to be better engaged in the through hole 111 of the clip 11.

It should be noted that, in the present embodiment, the distal end portion of the release member 131 further may be provided in other structures, and the other structures of the distal end portion of the release member 131 are not enumerated herein, as long as the other structures of the distal end portion of the release member 131 can make the distal end portion of the release member 131 engaged in the through hole 111 of the clip 11, and can drive the through hole 111 of the clip 11 when the control wire 13 is rotated.

Referring to FIG. 2 and FIG. 16, in the present embodiment, the support seat 15 is provided with a limit portion (position limiting portion), and the limit portion on the support seat 15 is configured to abut against the proximal end portion of the sleeve 12.

The limit portion on the support seat 15 can abut against the proximal end portion of the sleeve 12, so that when the sleeve 12 is mounted on the support seat 15, the sleeve can be mounted in a suitable position. Its function mainly lies in supporting the sleeve 12 when the control wire 13 is operated to pull the clip 11 proximally, until the release member 131 is pulled off from the through hole 111 of the clip 11, thereby ensuring the use effect of the tissue clamping and closing device for passing through an endoscope.

Referring to FIG. 16, the limit portion on the support seat 15 is a limit step.

Referring to FIG. 2, FIG. 7, and FIG. 8, in the present embodiment, an elastic barb 112 is provided at a position of each of two sides of the proximal end portion of the clip 11 close to the through hole 111, a locking slot 122 is provided on the proximal end portion of the sleeve 12, and the locking slot 122 is adapted to the elastic barb 112.

The configuration of the elastic barb 112 at the proximal end portion of the clip 11 and the locking slot 122 at the proximal end portion of the sleeve 12, when in use, can stably tighten the clip 11 in the sleeve 12 by the cooperation (engagement) between the locking slot 122 and the elastic barb 112.

Referring to FIG. 2, FIG. 7, and FIG. 8, in the present embodiment, optionally, there are multiple sets of locking slots 122 at the proximal end portion of the sleeve 12.

Providing multiple sets of locking slots 122 at the proximal end portion of the sleeve 12 can enable the sleeve 12 to be applied to other different clips 11, thereby improving the practicability of the tissue clamping and closing device for passing through an endoscope.

Referring to FIG. 2, FIG. 7, and FIG. 8, in the present embodiment, a guide long groove 123 is provided on the sleeve 12, the guide long groove 123 of the sleeve 12 is provided along a tightening direction of the clip 11, and the guide long groove 123 of the sleeve 12 is adapted to the elastic barb 112.

When the tissue clamping and closing device for passing through an endoscope is in use, the guide long groove 123 on the sleeve 12 can guide the tightening and opening of the clip 11, further ensuring the use stability and reliability of the tissue clamping and closing device for passing through an endoscope.

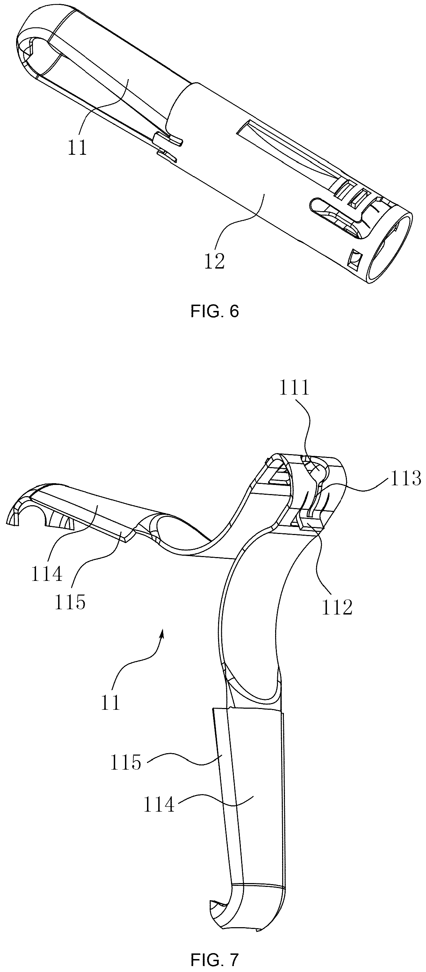

Referring to FIG. 2 and FIG. 7, in the present embodiment, expansion grooves 113 provided along a direction of the elastic barbs 112 are provided at two sides of the through hole 111 at the proximal end of the clip 11.

The configuration of the expansion groove 113 on the clip 11 can facilitate the release of the release member 131 from the through hole 111 of the clip 11 when the surgery is about to be completed, because the expansion groove 113 of the clip 11 will expand when a force is applied, thereby facilitating the release of the release member 131.

Referring to FIG. 2, FIG. 7, and FIG. 8, in the present embodiment, the clip 11 has clamping arm sections 114, and a blocking protrusion 115 extends outwardly from two sides of the clamping arm section 114 of the clip 11; a blocking groove 124 is provided at the distal end portion of the sleeve 12, and the blocking groove 124 at the distal end portion of the sleeve 12 is adapted to the blocking protrusion 115.

The blocking groove 124 at the distal end portion of the sleeve 12 will guide the blocking protrusion 115 on the clamping arm section 114 of the clip 11, so that the blocking protrusion 115 on the clamping arm section 114 of the clip 11 is tightened on the sleeve 12 following a track of the blocking groove 124 at the distal end portion of the sleeve 12, to prevent the tightened clip 11 from rotating randomly, further improving the use stability and reliability of the tissue clamping and closing device for passing through an endoscope.

It should be noted that, in another embodiment, the clip 11 and the sleeve 12 may also be in other structures, and other structures of the clip 11 and the sleeve 12 are not enumerated herein, as long as the clip 11 and the sleeve 12 in other structures can be applied to the tissue clamping and closing device for passing through an endoscope.

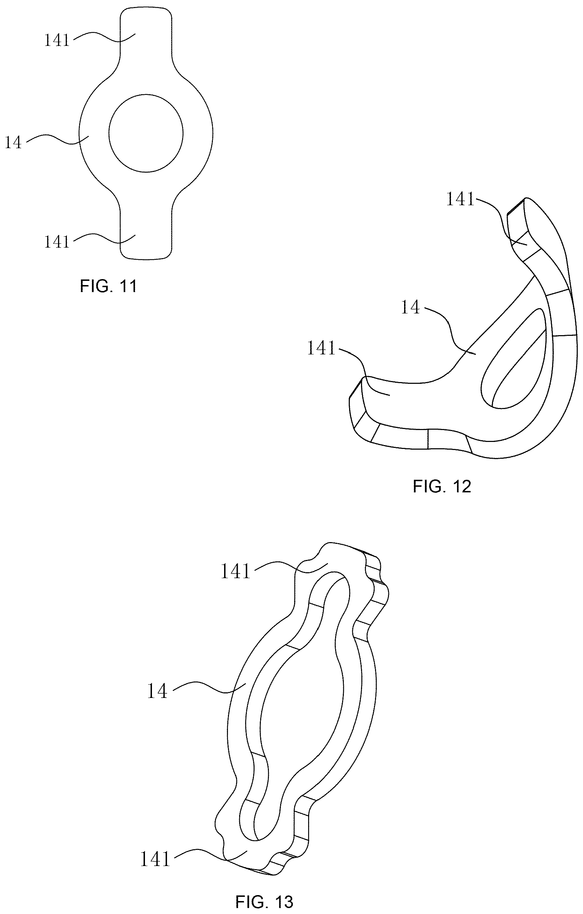

Referring to FIG. 11 and FIG. 12, as an optional embodiment, the specific structure of the elastic connecting piece 14 is as shown in FIG. 11 and FIG. 12, and after the clamping and closing assembly of the tissue clamping and closing device for passing through an endoscope is used to clamp and close tissues, the elastic connecting piece 14 will escape from the sleeve 12 of the hemostatic clip.

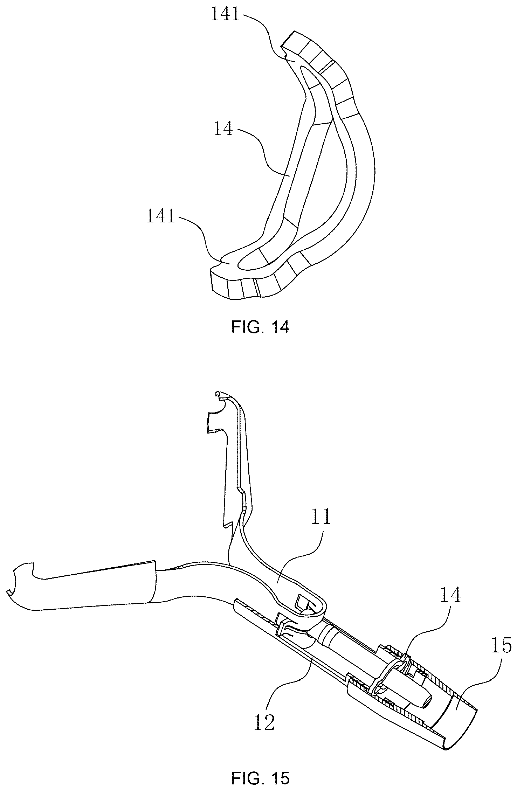

Referring to FIG. 13 to FIG. 15, as another optional embodiment, the specific structure of the elastic connecting piece 14 is as shown in FIG. 13 and FIG. 14, and after the clamping and closing assembly of the tissue clamping and closing device for passing through an endoscope clamps and closes tissues, the elastic connecting piece 14 will be left on the sleeve 12 of the hemostatic clip.

### Embodiment 2

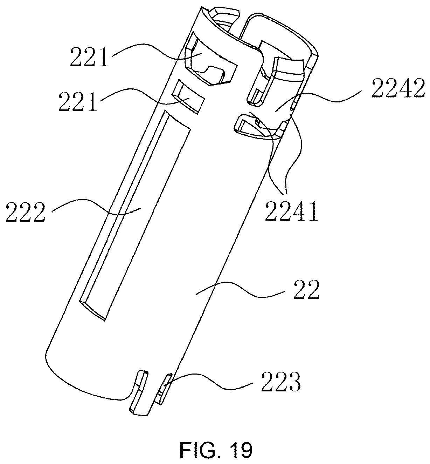

Referring to FIG. 1, FIG. 17 to FIG. 19, in the present embodiment, the tissue clamping and closing device for passing through an endoscope in the embodiment of the present disclosure is distinguished from that in Embodiment 1 in that:
the retainer is a torsion retaining piece 224 provided at a proximal end of a sleeve 22.

The torsion retaining piece 224 has a connecting portion 2241 and a torsion portion 2242, and the connecting portions 2241 are provided at two sides of the torsion portion 2242, and connected to the sleeve 22; the torsion portion 2242 is inclined, a proximal end portion of the torsion portion 2242 is inclined toward the inside of the sleeve 22, and a distal end portion of the torsion portion 2242 is inclined toward the outside of the sleeve 22.

The distal end portion of the torsion portion 2242 is configured to maintain the connection between the sleeve 22 and the axial sheath, and is configured to realize separation of the sleeve 22 from the axial sheath by deformation thereof under the action of the control wire 13.

In the present embodiment, the axial sheath includes a support seat 15 and a sheath body 16.

The sleeve 22 is detachably connected to a distal end of the support seat 15, a proximal end of the support seat 15 is connected to the distal end of the sheath body 16, and the control mechanism 17 is connected to the proximal end of the sheath body 16.

When the tissue clamping and closing device for passing through an endoscope in the embodiments of the present disclosure is in use, the distal end portion of the torsion portion 2242 is inclined toward the outside of the sleeve 22, and is snapped on the support seat 15. When the surgeon needs to clamp and close tissues, the surgeon can operate through the control mechanism 17 to pull back the control wire 13 connected with the control mechanism 17, the control wire 13 drives the clip 11 to move, and tightens the clip 11 in the sleeve 22, thus a clamping and closing operation is completed. When the clip 11 is tightened in the sleeve 22, the proximal end portion of the clip 11 will open the proximal end portion of the torsion portion 2242, so that inclination of the proximal end portion of the torsion portion 2242 toward the inside of the sleeve 22 turns to expansion toward the outside of the sleeve 22, further the inclination of the distal end portion of the torsion portion 2242 toward the outside of the sleeve 22 turns to contraction toward the inside of the sleeve 22, thus realizing the separation of the sleeve 22 from the support seat 15.

On the basis of ensuring that the tissue clamping and closing device for passing through an endoscope in the embodiments of the present disclosure has the rotating function and the function of opening and closing for many times, the structure of the tissue clamping and closing device for passing through an endoscope is greatly simplified, and its structure becomes quite simple, thus the production cost and the quality control cost are reduced, so that the use stability and reliability of the tissue clamping and closing device for passing through an endoscope are better guaranteed.

Referring to FIG. 17 to FIG. 19, similarly, in the present embodiment, a locking groove 221, a guide long groove 222, and a blocking groove 223 are provided on the sleeve 22.

Reference can be made to the contents of Embodiment 1 in the above for remaining contents of the embodiments of the present disclosure, and details are not described herein again in the present embodiment.

In all the above embodiments, "large" and "small" are relative, "more" and "less" are relative, "upper" and "lower" are relative, and expression of such relative terms is not repeated in the embodiments of the present disclosure.

It should be understood that reference to "in the present embodiment", "in the embodiment of the present disclosure" or "as an optional embodiment" throughout the specification means that particular features, structures or characteristics relating to the embodiment are included in at least one embodiment of the present disclosure. Therefore, "in the present embodiment", "in the embodiment of the present disclosure" or "as an optional embodiment" appearing throughout the specification does not necessarily refer to the same embodiment. Besides, these specific features, structures or characteristics may be incorporated in one or more embodiments in any suitable manner. It should also be understood by those skilled in the art that all the embodiments described in the specification belong to optional embodiments, and acts and modules involved are not necessarily required in the present disclosure.

In various embodiments of the present disclosure, it should be understood that the size of serial numbers of various processes in the above does not mean necessary execution order, and the execution order of various processes should be determined by its function and built-in logic, and should not limit the implementation process of the embodiments of the present disclosure.

The above-mentioned are merely embodiments of the present disclosure, but the scope of protection of the present disclosure is not limited thereto, and any modification or substitution that may be easily envisaged by those skilled in the present technical field within the technical scope disclosed in the present disclosure should fall within the scope of protection of the present disclosure. The scope of protection of the present invention is defined by the claims.

## Claims

1. A tissue clamping and closing device for passing through an endoscope, comprising:
a clip (11), wherein the clip (11) has at least two clip legs;
a control wire (13), wherein the control wire (13) is detachably connected to a through hole (111) at a proximal end of the clip (11);
an axial sheath, wherein the axial sheath is provided around the control wire (13);
a sleeve (22), wherein the sleeve (22) is configured to receive a proximal end portion of the clip (11) and is detachably connected to a distal end of the axial sheath;
a retainer; and
a control mechanism (17), wherein the control mechanism (17) is connected to a proximal end of the axial sheath and to a proximal end of the control wire (13),
wherein the control wire (13) is capable of controlling to open or close the at least two clip legs of the clip (11) by the control mechanism (17),
wherein the retainer is a torsion retaining piece (224), provided at a proximal end of the sleeve (22), **characterised in that** the torsion retaining piece (224) has connecting portions (2241) and a torsion portion (2242), and the connecting portions (2241) are provided at two sides of the torsion portion (2242), and connected to the sleeve; the torsion portion (2242) is inclined, a proximal end portion of the torsion portion (2242) is inclined toward inside of the sleeve (22), and a distal end portion of the torsion portion (2242) is inclined toward outside of the sleeve (22); and
the distal end portion of the torsion portion (2242) is configured to maintain a connection between the sleeve (22) and the axial sheath, and is configured to realize separation of the sleeve (22) from the axial sheath by deformation thereof under an action of the control wire (13).

2. The tissue clamping and closing device for passing through an endoscope according to claim 1, wherein the axial sheath comprises a support seat (15) and a sheath body (16), wherein
the sleeve (22) is detachably connected to a distal end of the support seat (15), a proximal end of the support seat (15) is connected to a distal end of the sheath body (16), and the control mechanism (17) is connected to a proximal end of the sheath body (16); and
a clamping groove (151) of the axial sheath is provided in the distal end of the support seat (15).

3. The tissue clamping and closing device for passing through an endoscope according to claim 2, wherein the support seat (15) is provided with a limit portion, and the limit portion in the support seat (15) is configured to abut against the proximal end of the sleeve (22).

4. The tissue clamping and closing device for passing through an endoscope according to claim 3, wherein the limit portion in the support seat (15) is a limit step.

5. The tissue clamping and closing device for passing through an endoscope according to claim 1, wherein an elastic barb (112) is provided at a position of each of two sides of the proximal end of the clip (11) close to the through hole (111), a locking slot (122) is provided on a proximal end of the sleeve (22), and the locking slot (122) is adapted to the elastic barb (112).

6. The tissue clamping and closing device for passing through an endoscope according to claim 5, wherein a guide long groove (222) is provided on the sleeve (22), the guide long groove (222) of the sleeve (22) is provided along a tightening direction of the clip (11), and the guide long groove (222) of the sleeve (22) is adapted to the elastic barb (112).

7. The tissue clamping and closing device for passing through an endoscope according to claim 5, wherein an expansion groove (113) extending towards the elastic barb (112) is provided at each of two sides of the through hole (111) at the proximal end of the clip (11).

8. The tissue clamping and closing device for passing through an endoscope according to claim 1, wherein the retainer is configured to realize separation of the sleeve (22) from the axial sheath by deformation thereof under an action of the control wire (13) and the clip (11).

## Patentansprüche

1. Gewebeklemm- und Verschlussvorrichtung zum Durchführen durch ein Endoskop, umfassend:
eine Klemme (11), wobei die Klemme (11) mindestens zwei Klemmenschenkel aufweist;
einen Steuerdraht (13), wobei der Steuerdraht (13) lösbar mit einem Durchgangsloch (111) an einem proximalen Ende der Klemme (11) verbunden ist;
eine axiale Hülle, wobei die axiale Hülle um den Steuerdraht (13) herum vorgesehen ist;
eine Hülse (22), wobei die Hülse (22) dazu ausgebildet ist, einen proximalen Endabschnitt der Klemme (11) aufzunehmen, und lösbar mit einem distalen Ende der axialen Hülle verbunden ist;
eine Halterung; und
einen Steuermechanismus (17), wobei der Steuermechanismus (17) mit einem proximalen Ende der axialen Hülle und mit einem proximalen Ende des Steuerdrahts (13) verbunden ist,
wobei der Steuerdraht (13) in der Lage ist, das Öffnen oder Schließen der mindestens zwei Klemmenschenkel der Klemme (11) durch den Steuermechanismus (17) zu steuern,
wobei die Halterung ein Torsionshaltestück (224) ist, das an einem proximalen Ende der Hülse (22) vorgesehen ist, **dadurch gekennzeichnet, dass**
das Torsionshaltestück (224) Verbindungsabschnitte (2241) und einen Torsionsabschnitt (2242) aufweist, und die Verbindungsabschnitte (2241) an zwei Seiten des Torsionsabschnitts (2242) vorgesehen und mit der Hülse verbunden sind; der Torsionsabschnitt (2242) geneigt ist, wobei ein proximaler Endabschnitt des Torsionsabschnitts (2242) zum Inneren der Hülse (22) hin geneigt ist und ein distaler Endabschnitt des Torsionsabschnitts (2242) zum Äußeren der Hülse (22) hin geneigt ist; und
der distale Endabschnitt des Torsionsabschnitts (2242) dazu ausgebildet ist, eine Verbindung zwischen der Hülse (22) und der axialen Hülle aufrechtzuerhalten, und dazu ausgebildet ist, eine Trennung der Hülse (22) von der axialen Hülle durch deren Verformung unter Einwirkung des Steuerdrahts (13) zu bewirken.

2. Gewebeklemm- und Verschlussvorrichtung zum Durchführen durch ein Endoskop nach Anspruch 1, wobei die axiale Hülle einen Stützsitz (15) und einen Hüllenkörper (16) umfasst, wobei die Hülse (22) lösbar mit einem distalen Ende des Stützsitzes (15) verbunden ist, ein proximales Ende des Stützsitzes (15) mit einem distalen Ende des Hülsenkörpers (16) verbunden ist und der Steuermechanismus (17) mit einem proximalen Ende des Hülsenkörpers (16) verbunden ist; und
eine Klemmnut (151) der axialen Hülse im distalen Ende des Stützsitzes (15) vorgesehen ist.

3. Gewebeklemm- und Verschlussvorrichtung zum Durchführen durch ein Endoskop nach Anspruch 2, wobei der Stützsitz (15) mit einem Begrenzungsabschnitt versehen ist und der Begrenzungsabschnitt im Stützsitz (15) dazu ausgebildet ist, am proximalen Ende der Hülse (22) anzuliegen.

4. Gewebeklemm- und Verschlussvorrichtung zum Durchführen durch ein Endoskop nach Anspruch 3, wobei der Begrenzungsabschnitt im Stützsitz (15) eine Begrenzungsstufe ist.

5. Gewebeklemm- und Verschlussvorrichtung zum Durchführen durch ein Endoskop nach Anspruch 1, wobei eine elastische Spitze (112) an einer Position an jeder der beiden Seiten des proximalen Endes der Klemme (11) nahe des Durchgangslochs (111) vorgesehen ist, ein Verriegelungsschlitz (122) an einem proximalen Ende der Hülse (22) vorgesehen ist und der Verriegelungsschlitz (122) an die elastische Spitze (112) angepasst ist.

6. Gewebeklemm- und Verschlussvorrichtung zum Durchführen durch ein Endoskop nach Anspruch 5, wobei eine lange Führungsnut (222) an der Hülse (22) vorgesehen ist, die lange Führungsnut (222) der Hülse (22) entlang einer Spannrichtung der Klemme (11) vorgesehen ist und die lange Führungsnut (222) der Hülse (22) an die elastische Spitze (112) angepasst ist.

7. Gewebeklemm- und Verschlussvorrichtung zum Durchführen durch ein Endoskop nach Anspruch 5, wobei eine sich in Richtung der elastischen Spitze (112) erstreckende Dehnungsnut (113) an jeder der beiden Seiten des Durchgangslochs (111) am proximalen Ende der Klemme (11) vorgesehen ist.

8. Gewebeklemm- und Verschlussvorrichtung zum Durchführen durch ein Endoskop nach Anspruch 1, wobei die Halterung dazu ausgebildet ist, eine Trennung der Hülse (22) von der axialen Hülle durch deren Verformung unter Einwirkung des Steuerdrahts (13) und der Klemme (11) zu bewirken.

## Revendications

1. Dispositif de serrage et de fermeture des tissus pour passer à travers un endoscope, comprenant :
une agrafe (11), dans lequel l'agrafe (11) a au moins deux pattes d'agrafe ;
un fil de commande (13), dans lequel le fil de commande (13) est relié de manière amovible à un trou traversant (111) à une extrémité proximale de l'agrafe (11) ;
une gaine axiale, dans laquelle la gaine axiale est prévue autour du fil de commande (13) ;
un manchon (22), dans lequel le manchon (22) est configuré pour recevoir une partie d'extrémité proximale de l'agrafe (11) et est relié de manière amovible à une extrémité distale de la gaine axiale ;
un dispositif de retenue ; et
un mécanisme de commande (17), dans lequel le mécanisme de commande (17) est relié à une extrémité proximale de la gaine axiale et à une extrémité proximale du fil de commande (13),
dans lequel le fil de commande (13) est capable de commander l'ouverture ou la fermeture des au moins deux pattes de l'agrafe (11) par le mécanisme de commande (17),
dans lequel le dispositif de retenue est une pièce de retenue de torsion (224), prévue à une extrémité proximale du manchon (22), **caractérisé en ce que**
la pièce de retenue de torsion (224) a des parties de liaison (2241) et une partie de torsion (2242), et les parties de liaison (2241) sont prévues sur les deux côtés de la partie de torsion (2242), et reliées au manchon ; la partie de torsion (2242) est inclinée, une partie d'extrémité proximale de la partie de torsion (2242) est inclinée vers l'intérieur du manchon (22), et une partie d'extrémité distale de la partie de torsion (2242) est inclinée vers l'extérieur du manchon (22) ; et la partie d'extrémité distale de la partie de torsion (2242) est configurée pour maintenir une liaison entre le manchon (22) et la gaine axiale, et est configurée pour réaliser la séparation du manchon (22) de la gaine axiale par déformation de celle-ci sous l'action du fil de commande (13).

2. Dispositif de serrage et de fermeture de tissu pour passer à travers un endoscope selon la revendication 1, dans lequel la gaine axiale comprend un siège de support (15) et un corps de gaine (16), dans lequel le manchon (22) est relié de manière amovible à une extrémité distale du siège de support (15), une extrémité proximale du siège de support (15) est reliée à une extrémité distale du corps de gaine (16), et le mécanisme de commande (17) est relié à une extrémité proximale du corps de gaine (16) ; et une rainure de serrage (151) de la gaine axiale est prévue dans l'extrémité distale du siège de support (15).

3. Dispositif de serrage et de fermeture de tissu pour passer à travers un endoscope selon la revendication 2, dans lequel le siège de support (15) est pourvu d'une partie limite, et la partie limite dans le siège de support (15) est configurée pour buter contre l'extrémité proximale du manchon (22).

4. Dispositif de serrage et de fermeture de tissu pour passer à travers un endoscope selon la revendication 3, dans lequel la partie limite dans le siège de support (15) est une étape limite.

5. Dispositif de serrage et de fermeture de tissu pour passer à travers un endoscope selon la revendication 1, dans lequel une barbe élastique (112) est prévue à une position de chacun des deux côtés de l'extrémité proximale de l'agrafe (11) à proximité du trou traversant (111), une fente de verrouillage (122) est prévue sur une extrémité proximale du manchon (22), et la fente de verrouillage (122) est adaptée à la barbe élastique (112).

6. Dispositif de serrage et de fermeture de tissu pour passer à travers un endoscope selon la revendication 5, dans lequel une longue rainure de guidage (222) est prévue sur le manchon (22), la longue rainure de guidage (222) du manchon (22) est prévue le long d'une direction de serrage de l'agrafe (11), et la longue rainure de guidage (222) du manchon (22) est adaptée à la barbe élastique (112).

7. Dispositif de serrage et de fermeture de tissu pour passer à travers un endoscope selon la revendication 5, dans lequel une rainure d'expansion (113) s'étendant vers la barbe élastique (112) est prévue sur chacun des deux côtés du trou traversant (111) à l'extrémité proximale de l'agrafe (11).

8. Dispositif de serrage et de fermeture de tissu pour passer à travers un endoscope selon la revendication 1, dans lequel le dispositif de retenue est configuré pour réaliser la séparation du manchon (22) de la gaine axiale par déformation de celle-ci sous l'action du fil de commande (13) et de l'agrafe (11).
